# EUROPEAN PATENT SPECIFICATION

(11) **EP 3 173 108 B1**
(45) Date of publication and mention of the grant of the patent: **26.09.2018**
(21) Application number: 15196991.2
(22) Date of filing: 30.11.2015
(51) Int. Cl.: A61M 1/10, A61M 1/12

(54) **IMPELLER WITH CLOSED CHANNELS FOR A CENTRIFUGAL IMPLANTABLE VENTRICULAR ASSIST PUMP**
LAUFRAD MIT GESCHLOSSENEN KANÄLEN FÜR EINE IMPLANTIERBARE VENTRIKULÄRE UNTERSTÜTZENDE ZENTRIFUGALPUMPE
ROTOR AVEC DES CANAUX FERMÉS POUR UNE POMPE D'ASSISTANCE VENTRICULAIRE CENTRIFUGE IMPLANTABLE

(43) Date of publication of application: 31.05.2017
(73) Proprietor: Fundacja Rozwoju Kardiochirurgii Im. Prof. Zbigniewa Religi, 41-800 Zabrze (PL)
(72) Inventor: KUSTOSZ, Roman, 41-800 Zabrze (PL); DAR AK, Maciej, 41-710 Ruda l ka (PL); ALTYNTSEV, Ievgenii, 40-024 Sumy (UA); SYNOWIEC, Tomasz, 44-100 Gliwice (PL); KORCZAK, Andrzej, 44-121 Gliwice (PL)
(74) Representative: Malcherek, Piotr

(56) References cited:
- WO-A1-2014/057087
- WO-A2-2011/117801
- AT-B- 394 136
- US-A- 5 290 236

## Description

### The invention

The present invention relates to an impeller with closed channels for a centrifugal implantable ventricular assist pump, provided for suspending without mechanical contact within a hybrid magnetic and hydraulic bearing of a ventricular assist pump.

### State of the art

WO2007/084339 discloses an impeller of a centrifugal blood pump placed in a blood pump chamber comprising an axial blood inlet and a tangential spiral channels leading to a blood outlet. Suspending the impeller during rotation within the pump chamber is performed by means of magnetic and hydrodynamic bearings. The impeller itself comprises a plurality of raised bodies formed by adjacent planes and curved surfaces of sidewalls. Said bodies are located at the periphery of the impeller and are spaced at a distance from one another. Each of said bodies is of similar shape and has an external wall, radially convex, constituting a portion of the periphery of said impeller, wherein the radius of convexity of the wall of said body is the same as the radius of the circle of the entire impeller. The planes are flat and formed by two straight sidewalls of unequal length. Said sidewalls extend to the interior of the impeller from the convex walls of the impeller periphery and intersect at an angle of approximately 90 degrees. In each body the volume thereof increases from the point of intersection of the straight sidewalls to the convex wall of the periphery. The edges of the intersecting sidewalls are rounded in order to reduce the risk of thrombosis and hemolysis. Between the raised bodies there are lowered surfaces creating channels for blood flow.

US2015/0010415 discloses a centrifugal blood pump having an impeller shaped as a disc with a centrally located through hole. The impeller is rotatably mounted in the blood pump chamber, and the centrally located hole is connected to a blood inlet, and on the circumference of the blood pump chamber there is a blood outlet. The impeller is located between two ring-shaped covers. On the surface of the impeller there are blades so that the surface of the impeller, of the blades and of the ring-shaped covers forms channels for blood flow. All blades on the surface of the impeller are of the same shape and are arranged at a regular angular distance from one another. The channels for blood flow are connected to the central hole of the impeller and run as far as the outer circumference of the impeller and their width increases along their length. Suspension of the impeller during rotation within the pump chamber is performed by means of magnetic and hydrodynamic bearings.

WO2014/179271 discloses an impeller of a centrifugal blood pump having a central hole maintained in a desired position within the pump chamber by means of magnetic and hydrodynamic bearings. The impeller consist of two ring-shaped discs. On the first of said discs there are blades, whereas the second disc constitutes a cover for covering the first disc with blades from the top. The blades and the internal surfaces of the discs form channels for blood running from the central hole of the impeller to the external circumferential edge thereof. See also WO2011/117801.

### The essence of the invention

The present invention relates to an impeller for a centrifugal implantable ventricular assist pump comprising a body, blades and closed interblade flow channels with a variable cross-section. The essence of the invention consists in that the blades of the impeller are of variable thickness and each channel is arch-shaped along the entire length thereof from the inlet to the outlet and said channel fulfils the following conditions:
- the angle of inflow onto the blade at the channel inlet ranges between 18° and 25°;
- the angle of outflow from the blade on the channel outlet is maximum 30°;
- cross-sections of the channel along the entire length from the inlet to the outlet are uniform in shape;
- the width and height of the channel increase continuously along the entire length thereof;
- the ratio of the width to the height of the channel increases continuously along the entire length thereof;
- the hydraulic radius of the channel increases continuously along the entire length thereof.

Specifically an outline of the cross-section of the channel is a rectangle with rounded corners.

The increase in height along the length of the channel at the outlet in relation to the height of the channel at the inlet ranges between 5 and 15 percent.

The increase in width along the length of the channel at the outlet in relation to the width of the channel at the inlet ranges between 5 and 20 percent.

The ratio of the width to the height of the channel along the length thereof ranges between 1.0 and 1.2.

The value of the hydraulic radius which increases continuously along the entire length of the channel ranges between 0.9 and 1.1 mm.

### Advantages of the invention

The impeller according to the invention is utilised in particular to forcing flow of blood in a rotary, centrifugal, implantable ventricular assist pump. The construction of the impeller ensures high efficiency of the pump, decreases shear stress and reduces hydraulic losses in the flow channels of said pump. Moreover, the construction of the impeller with a blade of variable thickness allows to locate construction elements of the contactless suspension of the impeller inside the blades of the impeller, which allows to decrease the height of the impeller and the weight thereof and thereby reduce the size of the entire pump, owing to which it is possible to implant said pump into a patient's body. Decreased overall size of the lateral surfaces of the impeller washed by blood reduces the risk of thrombosis within the pump. The construction of the channel with uniform cross-sections along the entire streamline and increasing surface results in reduced turbulences and elimination of points of stream break-up by the channel wall, thereby creating most advantageous for reducing shear stress produced on morphotic elements of blood flowing by the channel walls and for reducing activation of platelets. The flow of blood through channels is stable and orderly, and hydraulic friction of blood against the channel walls of the impeller has been substantially reduced. Increasing hydraulic radius while maintaining a uniform cross-section of the channel and velocity of the streamflow results in reduced hydraulic losses.

### Description of the drawings

The invention has been presented in greater detail in an embodiment illustrated in the enclosed drawing, where:

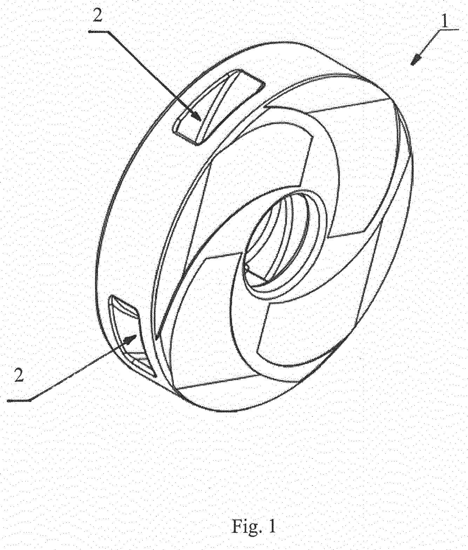

Fig. 1 - is a perspective view of the impeller,

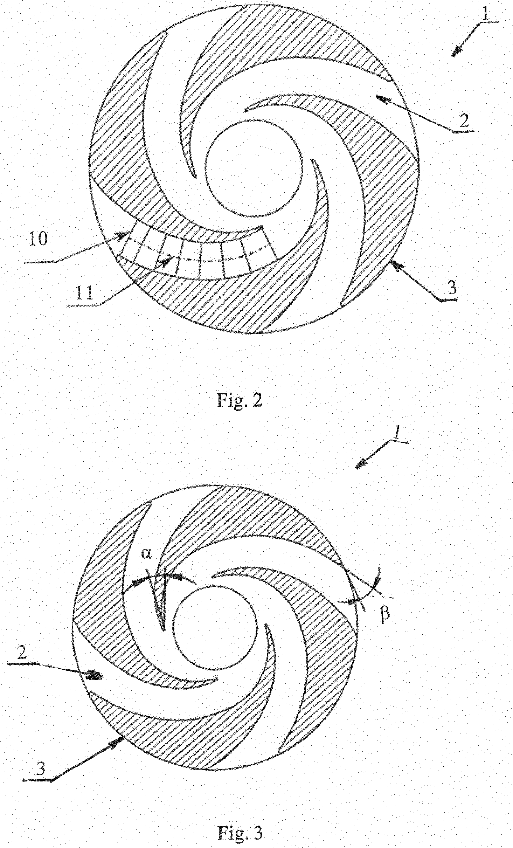

Fig. 2 - is a cross-section of the impeller with marked cross-sections of the interblade channel and middle streamline;
Fig. 3 - is a cross-section of the impeller with marked inlet and outlet angles of the blade;

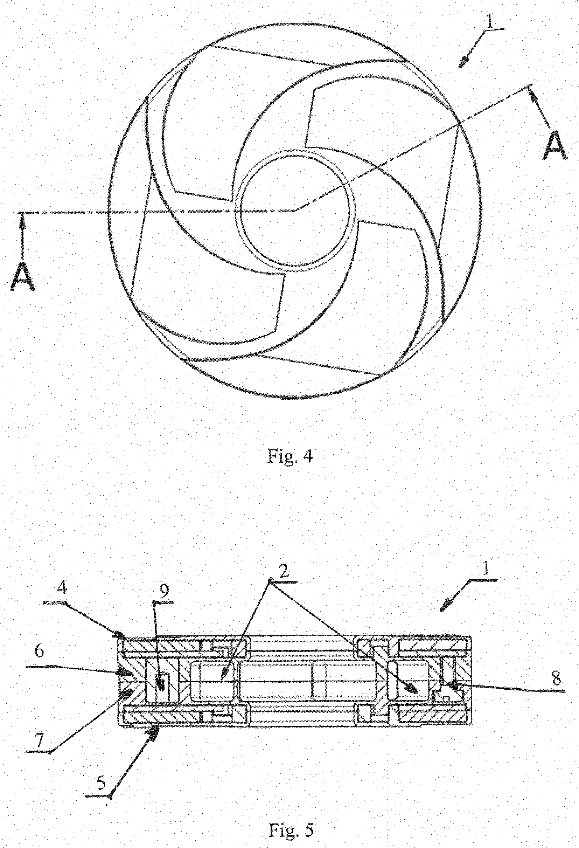

Fig. 4 - is a top view of the impeller;
Fig. 5 - is an A-A section of Fig. 4;

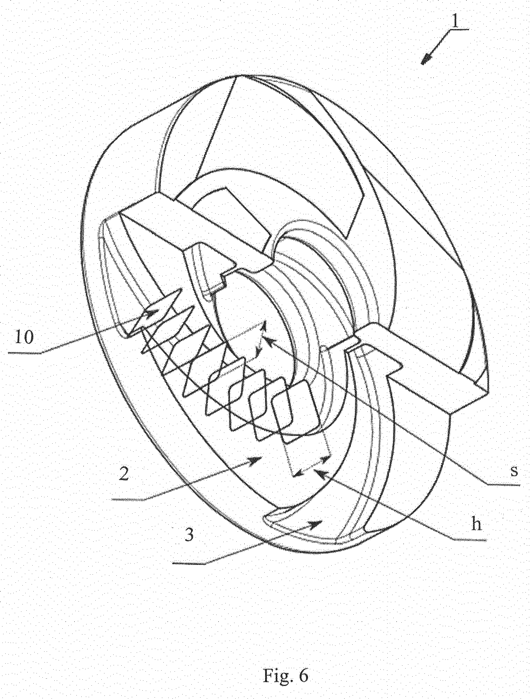

and Fig. 6 - is a partial perspective cross-section of the impeller.

### Embodiment

An impeller 1 is an element forcing flow in a rotary, centrifugal hydraulic pump, specifically in an implantable ventricular assist pump. The impeller 1 has four closed hydraulic, flow channels 2 separated by four blades 3.

The body of the impeller 1 comprises an upper shroud 4, a lower shroud 5, an upper core 6 and a lower core 7. The upper core 6 and the lower core 7 form the core of the impeller 1 with located therebetween closed flow channels 2. The upper core 6 and the lower core 7 are connected by means of screws 8 whose seats are located in the blades 3 of the lower core 7, and threaded holes are located in the blades 3 of the upper core 6. For the purpose of precise assembly of the above mentioned elements positioning pins 9 are placed in holes in the blades 3 of the upper core 6 and the lower core 7.

Drive magnets are radially arranged in the upper shroud 4 and the lower shroud 5 of the impeller 1. Magnetic fields of the magnets from the side of the flow channels are closed by means of a divider made from electrical steel sheets. Moreover, within the upper shroud 4 and the lower shroud 5 there are magnetic rings of a magnetic bearing. The shrouds 4, 5 of the impeller 1 with drive elements and magnetic suspension elements mounted therein are fixed to the core of the impeller 1. On the external surfaces of the lower shroud 5 and the upper shroud 4 there are elements of a hydraulic bearing.

Following assembly of the core of the impeller 1 the positioning pins 9 - depending on the need - may be utilised as elements for balancing the impeller 1 by proper sanding of said positioning pins 9 in order to reduce the weight thereof.

The impeller 1 is intended to be suspended in a hybrid contactless manner within the housing of the pump.

The blades 3 of the impeller 1 are of variable thickness, increasing radially outside the impeller 1. In cross-section of the impeller 1 lateral edges of the blades 3, between which the thickness of the impeller 1 is measured, are arch-shaped.

The interblade flow channels 2 are arch-shaped. The inflow angle α onto the blade at the channel 2 inlet ranges between 18° - 25°, whereas the outflow angle β from the blade at the channel 2 outlet does not exceed 30°. Cross-sections 10 of the channel 2 along the entire length thereof from the inlet to the outlet along the middle streamline 11 are uniform in shape, specifically an outline of a rectangle with rounded corners. The width s and the height h of the channel 2 along the entire length thereof increase continuously. Furthermore, the ratio of the width s to the height h of the channel 2 along the entire length thereof increases continuously, and the hydraulic radius of the channel 2 along the entire length thereof also increases continuously.

In particular an increase in height h of the channel 2 along the entire length thereof at the outlet in relation to the height h of the channel 2 at the inlet is not smaller than 5 % and not larger than 15 %, whereas an increase in the width s of the channel 2 along the entire length thereof at the outlet in relation to the width s of the channel 2 at the inlet is not smaller than 5 % and not larger than 20 %. The value of the ratio of the width s to the height h of the channel 2 ranges between 1.0 and 1.2.

The value of the hydraulic radius of the channel 2 increasing continuously along the entire length thereof ranges between 0.9 and 1.1 mm.

The tables below present exemplary dimensional parameters of the impeller:

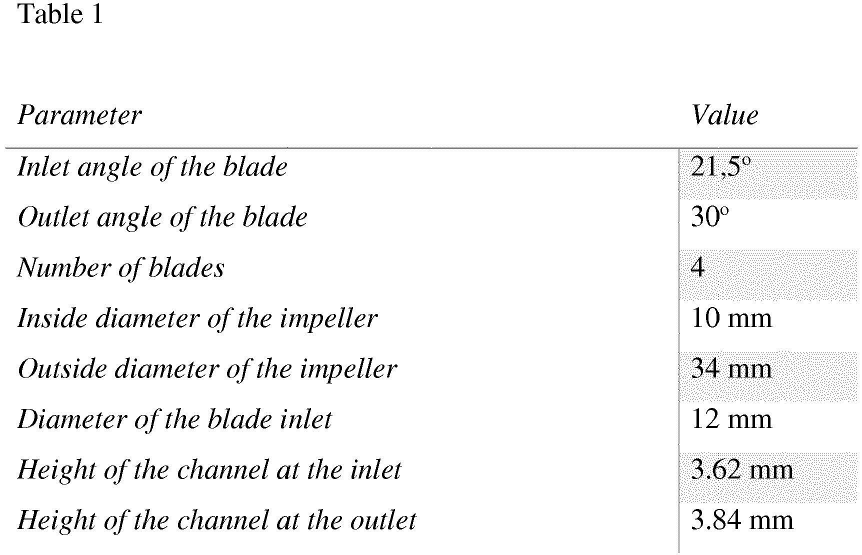

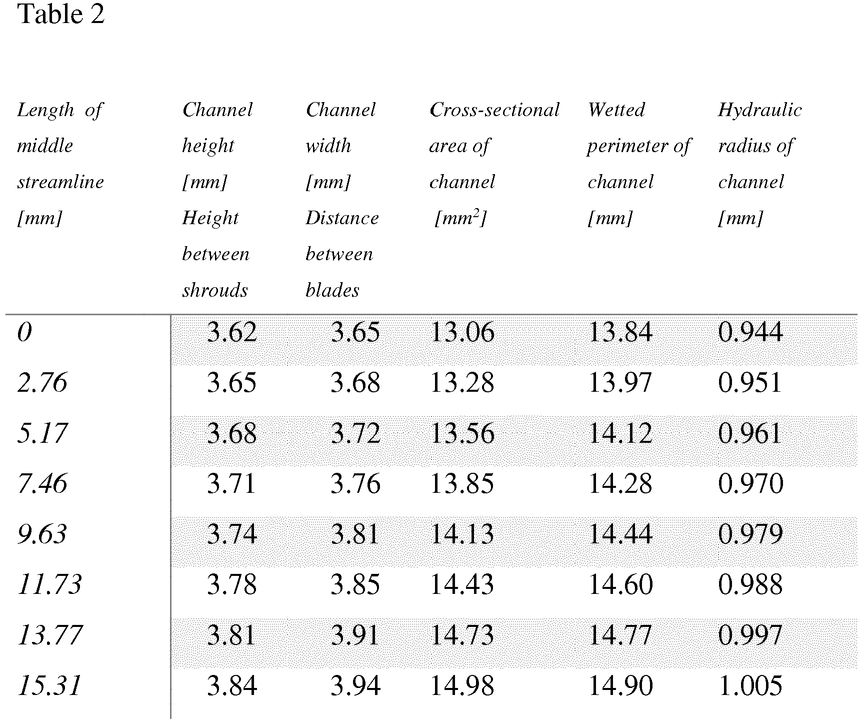

## Claims

1. An impeller (1) for a centrifugal implantable ventricular assist pump, comprising a body, blades (3) and closed interblade flow channels (2) of variable cross-sections, **characterised in that** the blades (3) of the impeller (1) are of variable thickness, and each channel (2) along the entire length thereof is arch-shaped and fulfils the following conditions:
- the inflow angle (α) onto the blade (3) at the inlet of the channel (2) ranges between 18° and 25°,
- the outflow angle (β) from the blade (3) at the outlet of the channel (2) is maximum 30°,
- cross-sections (10) of the channel (2) along the entire length thereof from the inlet to the outlet are uniform in shape;
- the width (s) and height (h) of the channel (2) along the entire length thereof from the inlet to the outlet increase continuously;
- the ratio of the width (s) to the height (h) of the channel (2) along the entire length thereof from the inlet to the outlet increases continuously;
- the hydraulic radius of the channel (2) along the entire length thereof from the inlet to the outlet increases continuously.

2. The impeller according to claim 1, **characterised in that** the cross-section of the channel (2) has a shape of a rectangle with rounded corners.

3. The impeller according to claim 1 or 2, **characterised in that** an increase in height (h) along the length of the channel (2) at the outlet in relation to the height (h) of the channel (2) at the inlet ranges between 5 and 15 %.

4. The impeller according to one of the claims 1 - 3, **characterised in that** an increase in the width (s) along the length of the channel (2) at the outlet in relation to the width (s) of the channel (2) at the inlet ranges between 5 and 20 %.

5. The impeller according to one of the claims 1 - 4, **characterised in that** the value of the ratio of the width (s) to the height (h) of the channel (2) along the length thereof from the inlet to the outlet ranges between 1.0 and 1.2.

6. The impeller according to one of the claims 1 - 5, **characterised in that** the value of the hydraulic radius of the channel (2) increasing continuously along the entire length thereof from the inlet to the outlet ranges between 0.9 and 1.1 mm.

## Patentansprüche

1. Ein Rotor (1) für eine zentrifugale implantierbare Herzunterstützungspumpe, umfassend ein Gehäuse, Blätter (3) und bedeckte interskapuläre Strömungskanäle (2) mit einem variablen Querschnitt, **dadurch gekennzeichnet, dass** die Blätter (3) des Rotors (1) eine variable Dicke aufweisen, und jeder Kanal (2) über seine gesamte Länge eine gewölbte Kurve aufweist und folgende Bedingungen erfüllt:
- der Einströmwinkel (α) zum Blatt (3) am Eintritt in den Kanal (2) liegt im Bereich von 18 ° bis 25 °;
- der Auslaufwinkel (β) vom Blatt (3) am Austritt aus dem Kanal (2) beträgt höchstens 30 °;
- die Querschnitte (10) des Kanals (2) entlang seines gesamten Verlaufs vom Eintritt zum Austritt haben die gleiche Form;
- die Breite (s) und Höhe (h) des Kanals (2) entlang seines gesamten Verlaufs vom Eintritt zum Austritt nehmen ständig zu;
- das Verhältnis der Breite (s) zur Höhe (h) des Kanals (2) entlang seines gesamten Verlaufs vom Eintritt zum Austritt nimmt ständig zu;
- der hydraulische Radius des Kanals (2) entlang seines gesamten Verlaufs vom Eintritt zum Austritt nimmt ständig zu.

2. Rotor nach Anspruch 1, **dadurch gekennzeichnet, dass** der Querschnitt des Kanals (2) ein rechteckiges Profil mit abgerundeten Ecken aufweist.

3. Rotor nach Anspruch 1 oder 2, **dadurch gekennzeichnet, dass** die Zunahme der Höhe (h) über die Länge des Kanals (2) am Austritt in Bezug auf die Höhe (h) des Kanals (2) am Einritt im Bereich von 5% -15% liegt.

4. Rotor nach einem der Ansprüche 1 - 3 **dadurch gekennzeichnet, dass** die Zunahme der Breite (s) über die Länge des Kanals (2) am Austritt in Bezug auf die Breite (s) des Kanals (2) am Einritt im Bereich von 5% - 20% liegt.

5. Rotor nach einem der Ansprüche 1 - 4 **dadurch gekennzeichnet, dass** der Wert des Verhältnisses der Breite (s) zur Höhe (h) des Kanals (2) entlang seiner Länge vom Eintritt zum Austritt im Bereich von 1,0 bis 1,2 liegt.

6. Rotor nach einem der Ansprüche 1 - 5 **dadurch gekennzeichnet, dass** der Wert des hydraulischen Radius des Kanals (2) auf seiner gesamten Länge vom Eintritt bis zum Austritt kontinuierlich zunimmt und im Bereich von 0,9 - 1,1 mm liegt.

## Revendications

1. Un rotor (1) pour une pompe implantable centrifuge assistant le coeur, comprenant un corps, des lames (3) et des canaux d'écoulement couverts, interlamellaires (2) avec une section transversale variable, **caractérisé en ce que** les lames (3) du rotor (1) ont une épaisseur variable, et chaque canal (2) a un trajet courbe sur toute leur longueur et remplit les conditions suivantes:
- l'angle d'entrée (α) sur la lame (3) à l'entrée du canal (2) se situe dans un intervalle allant de 18° à 25°,
- l'angle de sortie (β) de la lame (3) à la sortie du canal (2) a une valeur inférieure à 30°,
- les sections transversales (10) du canal (2) sur la totalité de son trajet à partir de l'entrée vers la sortie ont une forme identique;
- la largeur (s) et la hauteur (h) du canal (2) sur la totalité de son trajet à partir de l'entrée vers la sortie augmentent continuellement;
- le rapport entre la largeur (s) et la hauteur (h) du canal (2) sur la totalité de son trajet à partir de l'entrée vers la sortie augmentent continuellement;
- le rayon hydraulique du canal (2) sur la totalité de son trajet de l'entrée à la sortie est en constante augmentation.

2. Le rotor selon la revendication 1, **caractérisé en ce que** la section transversale du canal (2) présente un contour rectangulaire avec des coins arrondis.

3. Le rotor selon la revendication 1 ou 2, **caractérisé en ce que** l'augmentation de la hauteur (h) sur la longueur du canal (2) par rapport à la hauteur de sortie (h) du canal (2) à l'entrée se situe dans un intervalle de 5% - 15%.

4. Le rotor selon l'une des revendications de 1 - 3, **caractérisé en ce que** l'augmentation de la largeur (s) sur la longueur du canal (2) à la sortie par rapport à la largeur (s) du canal (2) à l'entrée se situe dans un intervalle de 5% - 20%.

5. Le rotor selon l'une des revendications 1-4, **caractérisé en ce que** le rapport entre la largeur (s) et la hauteur (h) du canal (2) sur sa longueur entre l'entrée et la sortie se situe dans un intervalle de 1,0 - 1,2.

6. Le rotor selon l'une des revendications 1-5, **caractérisé en ce que** la valeur du rayon hydraulique du canal (2) augmente de façon continue sur toute la longueur de son trajet depuis l'entrée jusqu'à la sortie dans un intervalle de 0,9 à 1,1 mm.
